# DEMANDE DE BREVET EUROPEEN

(11) **EP 1 319 663 A1**
(43) Date de publication de la demande: **18.06.2003**
(21) Numéro de dépôt: 01403267.6
(22) Date de dépôt: 17.12.2001
(51) Int. Cl.: C07F 1/02, C07D 475/04, C07C 229/24, A61K 31/4985, A61K 31/131, A61P 25/24

(54) **Sels de lithium d'acides folique, folinique et glutamique, antidépresseurs**

(71) Demandeur: Dospharma S.A., 76131 Mont Saint Aignan Cedex (FR)
(72) Inventeur: Rapin, Jean-Robet, 75014 Paris (FR)
(74) Mandataire: Burtin, Jean-François

(57) **Abrégé**

L'invention se rapporte au domaine de la chimie, et plus particulièrement au domaine de la chimie thérapeutique.

Elle a plus précisément pour objet de nouveaux sels dilithiés d'acide dicarboxylique choisis dans le groupe formé de l'acide folique, de l'acide folinique et de l'acide glutamique sous forme racémique, optiquement active ou sous forme d'isomères.

L'invention a également pour objet un procédé d'obtention des nouveaux sels dilithiés.

L'invention trouve son utilisation sous la forme de compositions pharmaceutiques associant le folate dilithié, le folinate dilithié et le glutamate dilithié avec un ou plusieurs excipients diluants ou adjuvants appropriés pour l'administration par voie orale, parentérale ou intra-veineuse.

## Description

L'invention s'adresse au domaine de la chimie thérapeutique et en particulier à des compositions pharmaceutiques à base de sels de lithium.

Un grand nombre de sels de lithium est déjà connu soit comme substituts des sels correspondants de sodium lorsque ceux-ci sont peu solubles (métasulfobenzoates de cortisoniques), soit lorsque le sel de sodium est trop hygroscopique. Cependant, la plupart du temps, les sels de lithium, d'acide minéral ou organique sont employés pour la présence de l'ion lithium, dont l'utilisation dans le traitement de la psychose maniaco-dépressive est un apport important dans la thérapeutique des maladies du comportement.

Les sels classiques tels que le carbonate, le gluconate, le citrate ou le bromure sont largement décrits dans la littérature. Leurs procédés d'obtention sont déjà mentionnés dans la littérature et les références qui s'y rapportent sont largement répertoriées dans The Merck Index, 12^{ème} édition, pages 943 à 945.
Cependant, l'utilisation des sels connus de lithium présente des inconvénients importants liés d'une part à la marge thérapeutique étroite de ces produits, et d'autre part à l'existence d'une compétition entre le lithium et le sodium lors de la réabsorption au niveau du tubule proximal, qui explique les grandes variations intra-individuelles, lorsqu'on modifie la diète sodée. En outre, l'administration de sels de lithium est source de carences en vitamines du groupe B et notamment en acide folique et ses dérivés.
Enfin, la biodisponibilité des sels de lithium est variable selon les sujets et reste toujours inférieure à 100 % d'où une grande difficulté à établir une posologie précise.

L'objet de la présente demande de brevet est en premier lieu d'offrir de nouveaux sels de lithium qui possèdent les qualités reconnues des sels antérieurs, mais qui ne présentent pas les inconvénients de ceux-ci précédemment exposés. En particulier, ces nouveaux sels n'entraînent pas les carences vitaminiques précitées ; qui plus est, les nouveaux sels selon l'invention manifestent un effet anti-dépressif plus puissant, ce qui permet de diminuer la posologie pour obtenir un effet comparable. Il en résulte une marge thérapeutique sensiblement élargie.
Le principe général sur lequel repose la présente invention est de trouver et de développer des sels qui permettent d'obtenir une lithémie suffisante sans avoir recours à des quantités de folate ou de folinate trop importantes pour permettre une administration efficace. L'invention consiste donc en l'association de deux nouveaux sels de lithium, le fol(in)ate de lithium et le glutamate de lithium.
Cette expression associe donc soit le folate de lithium soit le folinate de lithium et un précurseur de l'acide folique, à savoir le glutamate de lithium.
L'association folate de lithium / glutamate de lithium trouve un emploi thérapeutique dans le traitement de la dépression maniaco-dépressive chez le sujet jeune. L'association folinate de lithium / glutamate de lithium vise au même traitement chez le sujet âgé et pour des patients dont la transformation physiologique folate / folinate au niveau de l'intestin est altérée.

On entend par « folate de lithium » tout sel de lithium dérivé de l'acide folique, quelle que soit sa configuration racémique, optiquement active ou en mélange. Le terme « folinate de lithium » désigne tout sel de lithium de l'acide 5-formyl-5,6,7,8- tétrahydrofolique, quelle que soit sa configuration R, S ou SR, et quelle que soit sa conformation, droite, gauche ou racémique.
Le folate de lithium a pour formule : 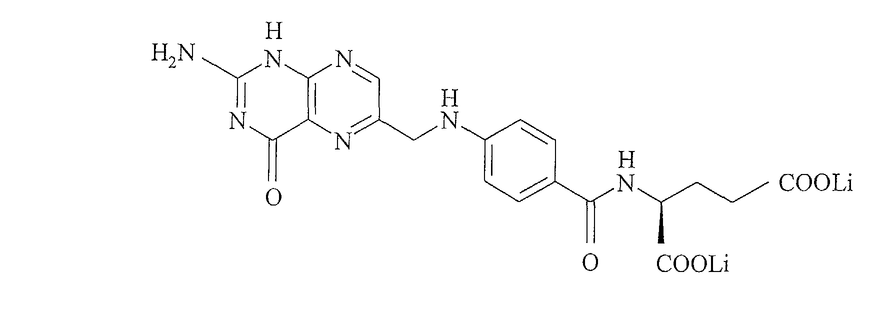 Le folinate de lithium a pour formule : 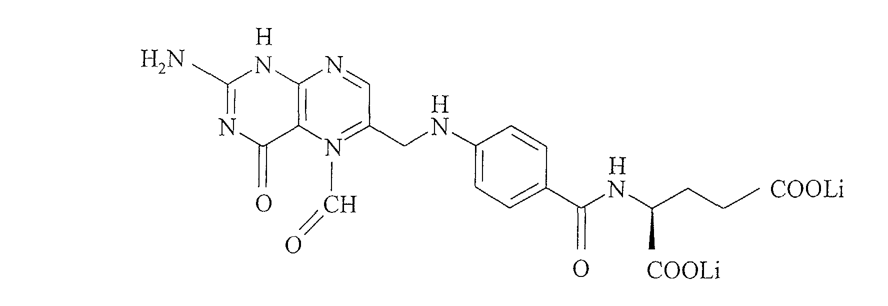 Le glutamate de lithium ou 2-aminopentane dicarboxylate de lithium a pour formule :

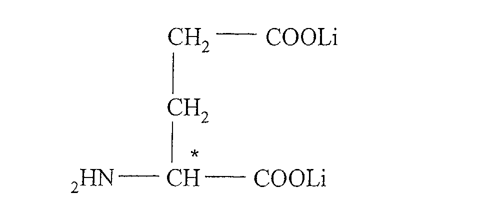

Il comporte un centre d'asymétrie qui peut être dédoublé en ses isomères d, l ou rester sous forme dl. La forme 1 est la forme naturelle.

L'invention se rapporte également à toute association d'un sel de lithium avec un acide minéral ou organique déjà décrit, avec un sel d'acide folique et/ou d'acide folinique déjà décrit tel que le folate de calcium ou le folinate de calcium.

L'invention se rapporte également à la préparation de nouveaux sels d'acide folique ou d'acide folinique optiquement actifs ou racémiques avec le lithium qui consiste à neutraliser une dispersion de préférence aqueuse d'acide folique ou d'acide folinique, avec une solution diluée d'un agent alcalin faiblement basique, en quantité bimolaire, jusqu'à obtention d'une solution limpide, addition d'une solution d'un sel de lithium préparée séparément dans la proportion d'un atome de lithium par reste carboxyle, on mélange les deux solutions aqueuses jusqu'à homogénéité et on concentre à sec dans des conditions ménagées jusqu'à obtention d'un résidu sec, reprise du résidu sec par un solvant organique, de préférence un solvant hydroxylé dans lequel les sels minéraux ne sont pas solubles.
La solution organique ainsi réalisée est évaporée à sec, on recueille les cristaux de folate de lithium ou de folinate de lithium ou de glutamate de lithium.
Ces nouveaux sels de lithium sont solubles dans l'eau et les solvants hydroxylés, notamment les alcools. Ces sels sont insolubles dans les solvants organiques inertes tels que le benzène ou le chlorure d'éthylène.
Les nouveaux sels de lithium selon l'invention sont caractérisés par leur poids moléculaire, leur point de fusion, l'analyse élémentaire, ainsi que par les données spectrales (UV, IR, RNM et pouvoir rotatoire). Ces données sont conformes à la structure attendue.
Le rendement d'obtention est pratiquement quantitatif.
Le glutamate de lithium est un autre objet de l'invention. La méthode de préparation est identique On obtient des cristaux rouge-orangé définis par leur point de fusion, le poids moléculaire et les données analytiques et spectrales.

L'invention a encore pour objet les compositions pharmaceutiques contenant au moins un des nouveaux sels de lithium définis ci-dessus, en association ou en mélange avec un ou plusieurs diluants, excipients ou adjuvants inertes non toxiques, thérapeutiquement compatibles.
De préférence, les compositions pharmaceutiques selon l'invention se présentent sous une des formes d'administration appropriée pour l'emploi par voie orale, parentérale ou intraveineuse.

Selon l'invention les compositions pharmaceutiques contiennent une quantité de principes actifs comme décrits ci-dessus, permettant de réaliser une posologie chez l'homme comprise entre 1 et 20 millimoles (mM) de lithium par jour. La lithémie efficace recommandée est de l'ordre de 0,5 à 0,8 mM/l quelle que soit la forme pharmaceutique administrée (forme à libération immédiate ou forme à libération prolongée). Cette lithémie est obtenue selon l'invention par administration de 10 à 20 mM de lithium soit l'équivalent de 150 à 300 mg de folate ou folinate de lithium.

Par ailleurs l'apport quotidien recommandé en acide folique ou en acide folinique étant de 5 mg, l'association de 5 mg de folate ou de folinate de lithium avec 160 mg de glutamate de lithium permet de respecter les apports quotidiens recommandés en lithium et en acide folique ou en acide folinique. L'apport en acide glutamique sous forme de glutamate de lithium est compatible avec les besoins journaliers pour cet acide aminé.
L'invention concerne encore un procédé de réalisation d'un médicament comportant une telle association pour l'obtention d'un médicament destiné au traitement des maladies dépressives, notamment de la dépression chez la personne âgée.
De préférence les compositions selon l'invention contiennent de 4 à 8 mg de folate de lithium ou de folinate de lithium et de 120 à 180 mg de glutamate de lithium par prise unitaire et de préférence 5 mg de folate ou de folinate de lithium et 150 mg à 160 mg de glutamate de lithium.
L'invention va être plus précisément expliquée à l'aide de la description détaillée et des exemples qui suivent sans toutefois y être limitée.

### EXEMPLE I

### Préparation du folate de lithium

On prépare une suspension de 4,10g d'acide folique (0,1 mol) dans 200ml d'eau distillée. Tout en maintenant la suspension sous agitation on ajoute une solution de 16,8g (0,2 mol) de bicarbonate de sodium dissous dans 200ml d'eau distillée. Après cessation de l'effervescence on obtient une solution limpide de folate disodique que l'on filtre.
On prépare séparément une solution de 5g de chlorure de lithium (0,2 mol) dissous dans 20ml d'eau distillée. On ajoute la solution de sel de lithium à la solution de folate disodique. On obtient une solution aqueuse de folate de lithium contenant en supplément du chlorure de sodium.

Par concentration sous vide au bain-marie bouillant, on obtient un résidu sec que l'on reprend à l'éthanol absolu. Le folate di lithien se dissout dans l'éthanol et on peut ainsi éliminer le chlorure de sodium par filtration ou centrifugation.

Après évaporation de l'éthanol on recueille des cristaux rouges de folate de lithium. Les cristaux sont solubles dans l'eau et les solvants hydroxylés.
Poids moléculaire 435,28
Point de fusion 300°C
Analyse élémentaire en accord avec la formule.
Le rendement est quantitatif.

### EXEMPLE II

La préparation du folinate de lithium s'effectue selon une méthode identique. Le folinate lithié se présente sous forme de cristaux rouges solubles dans l'eau et les alcools, son poids moléculaire est de 480,45, le point de fusion est de 305°C

Les analyses et les données spectrales confirment la structure du folinate dilithié.

### EXEMPLE III

On obtient le glutamate de lithium selon une méthode analogue. On obtient un sel sous forme de cristaux rouge orangé. Le poids moléculaire est de 159. Le point de fusion est de 215°C.
Les analyses et les données spectrales confirment la structure du nouveau sel.

### EXEMPLE IV

### Etude pharmacologique de l'association selon l'invention

On a procédé à l'expérimentation sur l'animal d'une association de 5 mg de folate dilithié et de 150 mg de glutamate de lithium ce qui correspond à la forme thérapeutique envisagée.

Du point de vue toxicité aigüe la DL₅₀ déterminée par voie orale chez la souris a été trouvée égale à 45 mg/Kg pour le mélange. Cette toxicité est nettement inférieure à celle propre au lithium seul, en calculant les correspondances pour le seul cation Il apparaît que l'ion folate et que l'ion glutamate diminuent la toxicité du lithium.

La biodisponibilité de l'association a été étudiée chez le rat. Le lithium administré sous forme de folate et de glutamate manifeste un taux élevé de résorption par la voie digestive. Le pic de concentration est observé 30 minutes après l'administration ce qui est plus rapide qu'avec le carbonate de lithium et la biodisponibilité est de 100 % ce qui est meilleur qu'avec les sels de lithium déjà décrits.

L'activité antidépressive du mélange folate de lithium - glutamate de lithium a été étudiée sur deux modèles de rats rendus dépressifs. On a utilisé comme molécule de référence, la desméthylimipramine (DMI) et comme produit de comparaison un mélange de carbonate de lithium, d'acide folique et d'acide glutamique dans les mêmes proportions que l'association selon l'invention. Les traitements ont été poursuivis per os avec administration biquotidienne durant huit jours.

### a) Premier modèle

Le premier modèle utilisé est le test du désespoir chez le rat (Despair Test). Les rats sont placés pour ce test dans une cuve d'eau tiédie à 27°C sans possibilité de s'échapper. Après une nage de quelques minutes, les animaux s'immobilisent et le temps moyen d'immobilité est chronométré. Il correspond à la phase de dépression. La DMI (antidépresseur de référence) à la dose de 10 mg diminue de 50 % ce temps d'immobilisation. Une courbe effet-dose a été établie avec l'association folate de lithium-glutamate de lithium. Un effet est observé dès la dose de 1 mg/kg. Il est maximum avec une dose de 20 mg/kg. A titre de comparaison, le lithium administré, sous forme de carbonate de lithium avec un mélange d'acide folique et d'acide glutamique, est moins actif sur ce modèle. On constate une potentialisation des effets du lithium lorsqu'il est administré sous forme de folate de lithium et de glutamate de lithium.

### b) Deuxième modèle

Le second test pratiqué est un test à voie unique (one-way)modifié. Dans un premier temps on apprend aux rats à passer d'un compartiment à un autre pour éviter un choc électrique. Les rats sélectionnés qui donnent systématiquement la réponse d'évitement sont placés dans un système à quatre compartiments avec retour au premier compartiment. Les animaux apprennent à éviter le choc électrique en passant du compartiment I à II, puis II à III et de II à IV. Le passage de IV vers I pose un problème puisque les rats reconnaissent (ou sentent) le point de départ.

Les rats s'immobilisent en position de « freezing » en dépit des décharges électriques qu'ils reçoivent. Le temps requis pour amener l'immobilisation correspond à une phase de dépression. La dose de 1 mg/kg de DMI supprime complètement ce temps d'immobilisation et ce, selon une relation dose/effet comprise entre 1 et 10mg/kg.

La simple association carbonate de lithium : acide folique et acide glutamique se montre moitié moins active en se rapportant à la dose de cation lithium administrée.
Les résultats pharmacologiques montrent que l'association folate de lithium à un glutamate de lithium est nettement active sur des modèles de dépression chez le rat, que cette activité est plus forte que celle des acides et du lithium pris simultanément, mais non sous forme de sels. On montre également que l'activité de l'association est plus élevée que celle du produit de référence (DMI).

Selon l'invention les compositions pharmaceutiques sont présentées en vue de l'administration orale, parentérale ou intraveineuse à une posologie active pour traiter soit la dépression soit la psychose maniaco-dépressive. Elle sera comprise entre 20 et 200 mg par jour en une ou plusieurs prises. Le rapport optimal entre le folate de lithium (ou le folinate de lithium) et le glutamate de lithium doit être compris entre 2 et 15 pour cent et de préférence entre 3 et 10 pour cent rapporté à la composition totale.

## Revendications

1. Sels de lithium d'acides dicarboxyliques choisis parmi l'acide folique, l'acide folinique et l'acide glutamique.

2. Disels de lithium des acides dicarboxyliques selon la revendication 1.

3. Folate di lithien selon la revendication 1.

4. Folinate di lithien selon la revendication 1.

5. Glutamate di lithien selon la revendication 1.

6. Composés selon l'une des revendications 1 à 5 sous forme optiquement active, sous forme racémique ou sous forme de stéréoisomères.

7. Compositions pharmaceutiques **caractérisées en ce qu'**elles renferment en association les composés dilithiés selon la revendication 1, en quantité suffisante et nécessaire pour assurer un apport biologique en lithium compatible avec les doses tolérables en acide folique et/ou en acide folinique.

8. Compositions pharmaceutiques selon la revendication 7, **caractérisées en ce qu'**elles se présentent sous une forme appropriée pour l'administration par voie orale, parentérale ou intraveineuse.

9. Compositions pharmaceutiques selon l'une des revendications 7 et 8, **caractérisées en ce qu'**elles renferment, à titre de principes actifs, une quantité de sels dilithiés selon la revendication 1 assurant une posologie journalière chez l'homme comprise entre 20 et 200 mg administrée en une ou plusieurs prises.

10. Compositions pharmaceutiques selon l'une des revendications 7 à 9 **caractérisées en ce qu'**elles contiennent de 4 à 8 mg de folinate ou de folate de lithium et de 120 à 180 mg de glutamate de lithium.

11. Procédé d'obtention d'un des sels dilithiés selon l'une des revendications 1 et 2, dans lequel on neutralise une dispersion, de préférence aqueuse, d'acide folique ou d'acide folinique ou d'acide glutamique avec une solution diluée d'un agent alcalin faiblement basique jusqu'à obtention d'une solution limpide, addition d'une solution de sel de lithium préparée séparément dans la proportion d'un atome de lithium par reste carboxyle, mélange des deux solutions aqueuses jusqu'à homogénéité, concentration à sec dans des conditions ménagées jusqu'à obtention d'un résidu sec que l'on reprend par un solvant organique, sépare la phase organique, l'évaporé à sec et recueille les cristaux de folate de lithium ou le folinate de lithium ou de glutamate de lithium.
